# EUROPEAN PATENT SPECIFICATION

(11) **EP 1 140 122 B1**
(45) Date of publication and mention of the grant of the patent: **26.05.2004**
(21) Application number: 00900287.4
(22) Date of filing: 14.01.2000
(51) Int. Cl.: A61K 35/56, A61P 29/00, A61P 35/00

(54) **PRO-APOPTOTIC AGENTS**
PRO-APOPTOTISCHE STOFFE
AGENTS FAVORISANT L'APOPTOSE

(30) Priority: 15.01.1999 GB 9900930
(43) Date of publication of application: 10.10.2001
(73) Proprietor: THE UNIVERSITY OF NOTTINGHAM, Nottingham NG7 2RD (GB)
(72) Inventor: CHOW, Sek, Chuen, Nottingham NG7 2RD (GB); PRITCHARD, David, Idris, Nottingham NG7 2RD (GB)
(74) Representative: Perry, Robert Edward
(86) International application number: PCT/GB2000/000090
(87) International publication number: WO 2000/041706

(56) References cited:
- WO-A-95/12615
- BROPHY ET AL.: "Glutathione S-transferase (GST) expression in the human hookworm Necator americanus: potential roles for excretory-secretory forms of GST" ACTA TROPICA, vol. 59, 1995, page 259-263 XP000881639
- KALINKOVICZH ET AL.: "Decreased CD4 and increased CD8 counts with T-Cell activation is associated with chronic helminth infection" CLIN. EXP. IMMUNOLOGY, vol. 114, no. 3, December 1998 (1998-12), pages 414-421, XP000881640
- BEHNKE ET AL.: "Resistance of the hookworms Ancylostoma ceylanicum and Necator americanus to intestinal inflammatory responses induced by heterologous infection" INT. J. PARASITOLOGY, vol. 24, no. 1, 1994, pages 91-101, XP000881612

## Description

### Field of the Invention

This invention relates to pro-apoptotic agents isolatable from Necator *americanus.*

### Background to the Invention

Human nematodes (roundworms) include the hookworm nematode species, *Necator americanus.* Adult females of *N. americanus* are typically 9-11 mm in length and adult males are typically 7-9 mm in length. These adult worms commonly reside in the lumen of the small intestine, and attach to the intestinal wall resulting in blood loss from the host. Eggs are passed out in the faeces and, under favourable conditions, usually hatch in 1-2 days. Larvae are then released and continue to grow in the faeces and/or the soil. After up to 10 days, the larvae are infectious, and may survive 3-4 weeks in this condition. If, during this time, contact is made with a human host, the larvae can penetrate the skin, after which they may be carried through the veins and the heart to the lungs. Here, they penetrate the pulmonary alveolae and ascend the bronchial tree to the pharynx where they can be swallowed and delivered to the small intestine. They then develop into adult worms. Typically, six weeks or more is required from the initial infection to oviposition by the adult female.

*N. americanus* is found in tropical and sub-tropical localities, where it gives rise to a hookworm disease having a number of clinical features. Iron deficiency anaemia, resulting from blood loss at the site of intestinal attachment of the adult worms, is the most common symptom of hookworm infection, and may be accompanied by cardiac complications. Gastrointestinal and nutritional/metabolic symptoms may also be found. Additionally, itching may occur during the initial infection, and respiratory symptoms may be observed during the pulmonary migration stage.

Apoptosis is a suicide process built into all mammalian cells in which a cell dies in a controlled manner. Cells undergoing apoptosis show distinctive morphological changes, for instance nuclear condensation and the formation of apoptotic bodies. The biochemical hallmark of apoptosis is the cleavage of chromatin into nucleosomal fragments.

### Summary of the invention

The present invention is based on the realisation that hookworms shield against immunological attack by producing a factor capable of reducing the viability of reactive T cells. This factor may therefore exert an effect that results in cell apoptosis and may have valuable therapeutic application.

The present invention therefore provides a substantially pure excretory-secretory (ES) product, isolatable from *N. americanus,* and functional derivatives thereof, capable of reducing cell viability. Cell viability may be reduced via the induction of apoptosis.

The product of the invention is a protein of less than 12kDa, or a functional fragment thereof.

The invention further provides a use for this protein, in the manufacture of a pro-apoptotic composition.

The invention further provides a pro-apoptotic composition comprising a pharmaceutically-acceptable diluent or carrier, and this protein.

The invention further provides the use of this protein in the manufacture of a medicament with anti-tumour and/or anti-inflammatory activity, ie for the treatment of cancer or an inflammation disorder.

### Brief Description of the Drawings

In the drawings:
Figure 1 shows the effect of *N. americanus* excretory-secretary products on the cell viability of human leukaemic T-cell line Jurkat, where (X) represents protein concentration (*µ*g/ml) and (Y) represents the percentage cell viability; and
Figure 2 shows the effect of partially purified excretory-secretory products on the cell viability of the human leukaemic T-cell line Jurkat, where (X) represents cell fractions and (Y) represents the cell viability index.

### Description of the Invention

By way of example only, excretory-secretory (ES) products of *N. americanus* may be prepared in the following manner.

*Necator americanus* is passaged in DSN hamsters. Faecal culture from the infected animals provide infective larvae, which are then used to infect neonates percutaneously. Adult worms are routinely harvested from the small intestine of infected hamsters 5 weeks post-infection. The ileum of the infected hamster is removed, opened longitudinally, and placed in Hanks' saline at 37°C. As worms release their hold on the mucosa, they are carefully removed, thoroughly washed, and cleansed in Hanks' saline containing 100 IU/ml penicillin and 100 *µ*g/ml streptomycin. Cleansed worms are examined under a dissecting microscope, and undamaged worms retained.

Under sterile conditions, worms are added to RPMI 1640, containing penicillin and streptomycin, as above. The worms are then cultured for 16 hours, and the supernatants removed for analysis of pro-apoptotic activities.

Cultured supernatants are sterile-filtered through 0.2 *µ*m filters, which also removes eggs that may have deposited during the culture period.

Protein concentration of the supernatants is assayed using Coomasie Brilliant Blue with BSA as standards.

To assess the effects of hookworm ES on the viability of Jurkat cells, 2 x 10⁵ cells were cultured with various concentration of ES products in a final volume of 200 *µ*l in flat-bottomed 96-well plates for 16 hours at 37°C in a 5% CO₂ incubator. This was followed by the addition of 20 *µ*l of Thiazol blue solution (5 mg/ml) to the cells and the plates were incubated for a further 4 hours. After the incubation, 150 *µ*l of medium was removed carefully from the wells, followed by the addition of 150 *µ*l iso-propanol, and mixed thoroughly. The OD at 590 and 650 nm was determined on an ELISA reader. Cell viability was expressed as the percentage of control absorbance obtained in untreated cells after subtracting the absorbance from appropriate blanks.

The induction of apoptosis in Jurkat T-cells by ES products was monitored by staining fixed cells with Hoechst dye 33358 (50 *µ*g/ml in PBS) and examining the nuclear morphological changes using confocal laser microscopy, and the analysis of oligonucleosomal DNA fragments in the Jurkat cells using agarose gel electrophoresis.

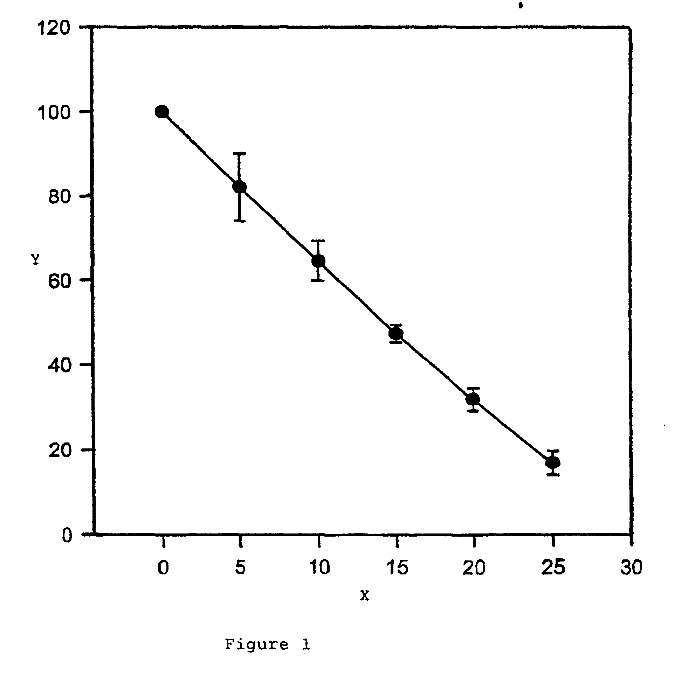

Figure 1 shows the effect of *Necator americanus* ES products on Jurkat cell viability. Cell viability was reduced (ie cells were killed) in a dose-dependent manner. Cell viability was shown to be reduced via the induction of apoptosis. The characteristic cleavage of chromatin into nucleosomal fragments, that is indicative of apoptosis, was observed. A further characteristic of apoptosis is the change in nuclear morphology and this was also observed in the cells after treatment with ES products.

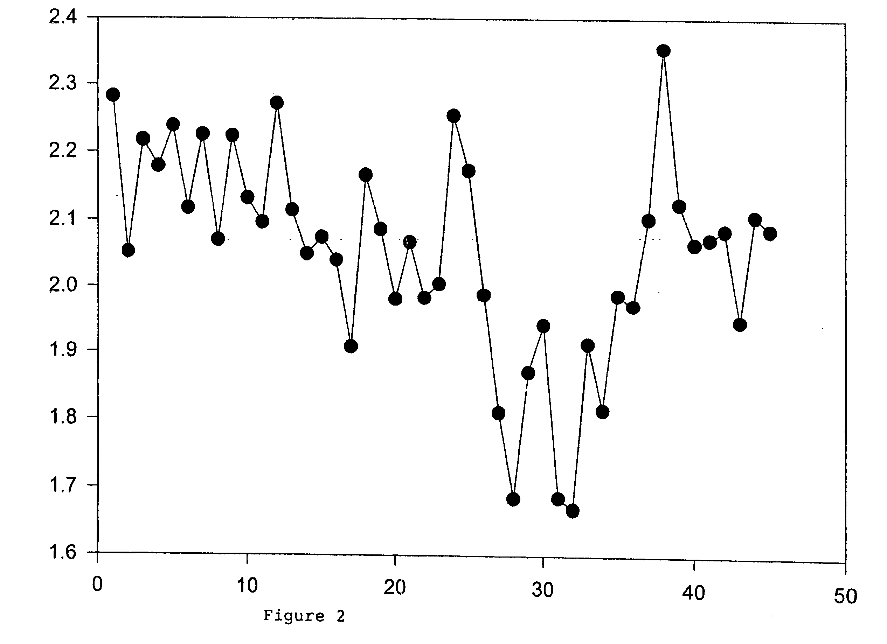

After fractionation through a Sephacryl S-300 column, the fractionated *N. americanus* preparation was assessed for pro-apoptotic activity. Each fraction was then co-cultured with Jurkat cells, and the cell viability index determined. Values of less than 1.0 indicate apoptotic cells. Figure 2 shows the cell viability index of fractions 1 to 45. Fractions 27-33 were found to have significantly lower cell viability indexes (<1.9) and therefore cell killing activities. Subsequent incubation of Jurkat cells with these fractions induced apoptosis in the cells. Fractions 27-33 were concentrated and separated on a 15% SDS PAGE. The gel showed very little protein bands but indicated that the pro-apoptotic agent may be less than 12 kDA in size.

The product of the invention may be formulated into a composition for therapeutic application. Suitable formulations will be apparent to the skilled person, including acceptable excipients and diluents. The product may also be formulated with a carrier which targets a particular site in vivo, e.g. to a tumour.

## Claims

1. A protein of less than 12 kDa, or a functional fragment thereof, and being capable of inducing apoptosis in reactive T-cells, the protein being obtainable by fractionating a supernatant containing the secretory-excretory products of *Necator Americanus.*

2. A protein according to claim 1, for use in therapy.

3. A pro-apoptotic composition comprising a pharmaceutically acceptable diluent or carrier, and a protein as defined in either preceding claim.

4. The use of a protein as defined in any preceding claim, in the manufacture of a medicament for the treatment of cancer.

5. The use of a protein as defined in any of claims 1 to 3, in the manufacture of a medicament for the treatment of an inflammatory disease.

## Patentansprüche

1. Protein von weniger als 12 kDa oder ein funktionales Fragment hiervon, und mit der Fähigkeit Apoptose in reaktiven T-Zellen zu induzieren, wobei das Protein erhältlich ist durch Fraktionieren eines Überstandes, der sekretorischexkretorische Produkte von *Necator americanus* enthält.

2. Protein nach Anspruch 1 für die Verwendung in der Therapie.

3. Pro-apoptotische Zusammensetzung, umfassend ein(en) pharmazeutisch annehmbares(n) Verdünnungsmittel oder Träger, und ein Protein, wie in einem der vorangehenden Ansprüche definiert.

4. Verwendung eines Proteins, wie in irgendeinem der vorangehenden Ansprüche definiert, bei der Herstellung eines Medikaments zur Krebsbehandlung.

5. Verwendung eines Proteins, wie in irgendeinem der Ansprüche 1 bis 3 definiert, bei der Herstellung eines Medikaments für die Behandlung einer entzündlichen Erkrankung.

## Revendications

1. Protéine de moins de 12 kDa, ou un de ses fragments fonctionnels, capable d'induire l'apoptose dans des lymphocytes T réactifs, la protéine pouvant être obtenue par fractionnement d'un surnageant contenant les produits de sécrétion-excrétion de *Necator americanus.*

2. Protéine selon la revendication 1, à utiliser en thérapie.

3. Composition favorisant l'apoptose, comprenant un diluant ou un support pharmaceutiquement acceptable et une protéine telle que définie dans l'une ou l'autre des revendications précédentes.

4. Utilisation d'une protéine telle que définie dans l'une quelconque des revendications précédentes dans la fabrication d'un médicament destiné au traitement du cancer.

5. Utilisation d'une protéine telle que définie dans l'une quelconque des revendications 1 à 3 dans la fabrication d'un médicament destiné au traitement d'une maladie inflammatoire.
